# EUROPEAN PATENT SPECIFICATION

(11) **EP 2 004 826 B1**
(45) Date of publication and mention of the grant of the patent: **02.03.2011**
(21) Application number: 07726885.2
(22) Date of filing: 14.03.2007
(51) Int. Cl.: C12N 15/80, C12N 15/90

(54) **IMPROVED METHOD FOR HOMOLOGOUS RECOMBINATION IN EUKARYOTIC CELLS**
VERBESSERTES VERFAHREN ZUR HOMOLOGEN REKOMBINATION IN EUKARYONTISCHEN ZELLEN
PROCÉDÉ AMÉLIORÉ DE RECOMBINAISON HOMOLOGUE DANS DES CELLULES EUCARYOTES

(30) Priority: 08.04.2006 EP 06075904
(43) Date of publication of application: 24.12.2008
(73) Proprietor: DSM IP Assets B.V., 6411 TE Heerlen (NL)
(72) Inventor: BERG, VAN DEN, Marco Alexander, NL-2685 EH Poeldijk (NL); KERKMAN, Richard, NL-2042 NL Zandvoort (NL); TOUW-RIEL, Hesselien, NL-2645 GC Delfgauw (NL)
(74) Representative: Matulewicz, Emil Rudolf Antonius
(86) International application number: PCT/EP2007/052392
(87) International publication number: WO 2007/115886

(56) References cited:
- WO-A2-02/052026
- US-B1- 6 204 061
- SHAKED HEZI ET AL: "High-frequency gene targeting in Arabidopsis plants expressing the yeast RAD54 gene" PROCEEDINGS OF THE NATIONAL ACADEMY OF SCIENCES OF USA, NATIONAL ACADEMY OF SCIENCE, WASHINGTON, DC, US, vol. 102, no. 34, August 2005 (2005-08), pages 12265-12269, XP009087433 ISSN: 0027-8424

### SEQUENCE LISTING

<110> DSM IP Assets B.V.
<120> IMPROVED METHOD FOR HOMOLOGOUS RECOMBINATION IN EUKARYOTIC CELLS
<130> 26061wO
<160> 50
<170> PatentIn version 3.1
<210> 1
   <211> 33
   <212> DNA
   <213> Artificial sequence
<220>
   <223> Description of artificial sequence: synthetic primer
<400> 1
   ttctctagaa acagctggca acgggcaatc tac 33
<210> 2
   <211> 46
   <212> DNA
   <213> Artificial sequence
<220>
   <223> Description of artificial sequence: synthetic primer
<900> 2
   cgaggatacc tacttcgtaa caacattgat ttgaagtttg ctcagg 46
<210> 3
   <211> 45
   <212> DNA
   <213> Artificial sequence
<220>
   <223> Description of artificial sequence: synthetic primer
<400> 3
   cctgagcaaa cttcaaatca atgttgttac gaagtaggta tcctc 45
<210> 4
   <211> 46
   <212> DNA
   <213> Artificial sequence
<220>
   <223> Description of artificial sequence: synthetic primer
<400> 4
   cggatgcggc cgctgcgccc gggccctcga gacgattacg cattcc 46
<210> 5
   <211> 88
   <212> DNA
   <213> Artificial sequence
<220>
   <223> Description of artificial sequence: synthetic primer
<400> 5
   ccagcggccg ccggcccggg cataacttcg tatagcatac attatacgaa gttatcctgc 60
   agggactctt tctggcatgc ggagagac 88
<210> 6
   <211> 43
   <212> DNA
   <213> Artificial sequence
<220>
   <223> Description of artificial sequence: synthetic primer
<400> 6
   attgcggccg cggggcgcgc cgttgagtgg tatggggcca tcc 43
<210> 7
   <211> 85
   <212> DNA
   <213> Artificial sequence
<220>
   <223> Description of artificial sequence: synthetic primer
<400> 7
   tcatggtacc agcccgggct ggcgcgccat aacttcgtat agcatacatt atacgaagtt 60
   atgttacgaa gtaggtatcc tcggg 85
<210> 8
   <211> 33
   <212> DNA
   <213> Artificial sequence
<220>
   <223> Description of artificial sequence: synthetic primer
<400> 8
   ctttggtacc cttttcggcg agataatctc gac 33
<210> 9
   <211> 58
   <212> DNA
   <213> Artificial sequence
<220>
   <223> Description of artificial sequence: synthetic primer
<400> 9
   gaacaaaagc ttggtggcgc gcccccggta ccggtcgact acatgtatct gcatgttg 58
<210> 10
   <211> 39
   <212> DNA
   <213> Artificial sequence
<220>
   <223> Description of artificial sequence: synthetic primer
<400> 10
   gaaaaggaaa agcggccgca ttaaagcctt cgagcgtcc 39
<210> 11
   <211> 21
   <212> DNA
   <213> Artificial sequence
<220>
   <223> Description of artificial sequence: synthetic primer
<400> 11
   gccacaggtg actctggatg g 21
<210> 12
   <211> 20
   <212> DNA
   <213> Artificial sequence
<220>
   <223> Description of artificial sequence: synthetic primer
<400> 12
   taacagggag ataggtgaag 20
<210> 13
   <211> 21
   <212> DNA
   <213> Artificial sequence
<220>
   <223> Description of artificial sequence: synthetic primer
<400> 13
   tgaagaagca cggctaattg g 21
<210> 14
   <211> 21
   <212> DNA
   <213> Artificial sequence
<220>
   <223> Description of artificial sequence: synthetic primer
<400> 14
   gtctctccgc atgccagaaa g 21
<210> 15
   <211> 20
   <212> DNA
   <213> Artificial sequence
<220>
   <223> Description of artificial sequence: synthetic primer
<400> 15
   atggttgaag atagctacac 20
<210> 16
   <211> 21
   <212> DNA
   <213> Artificial sequence
<220>
   <223> Description of artificial sequence: synthetic primer
<400> 16
   aaacttctgc tcaaagtact g 21
<210> 17
   <211> 21
   <212> DNA
   <213> Artificial sequence
<220>
   <223> Description of artificial sequence: synthetic primer
<400> 17
   ccaagacgat cctgagcaaa c 21
<210> 18
   <211> 20
   <212> DNA
   <213> Artificial sequence
<220>
   <223> Description of artificial sequence: synthetic primer
<400> 18
   ggtcaatgac gctccattgg 20
<210> 19
   <211> 33
   <212> DNA
   <213> Artificial sequence
<220>
   <223> Description of artificial sequence: synthetic primer
<400> 19
   caatctagac gacgatttga atgttttcca ccc 33
<210> 20
   <211> 47
   <212> DNA
   <213> Artificial sequence
<220>
   <223> Description of artificial sequence: synthetic primer
<400> 20
   gccattttcc caacatcatc atcattggaa ttgagtgggg gggcggg 47
<210> 21
   <211> 47
   <212> DNA
   <213> Artificial sequence
<220>
   <223> Description of artificial sequence: synthetic primer
<400> 21
   cccgcccccc cactcaattc caatgatgat gatgttggga aaatggg 47
<210> 22
   <211> 46
   <212> DNA
   <213> Artificial sequence
<220>
   <223> Description of artificial sequence: synthetic primer
<400> 22
   ctggcggccg cttagcccgg gctcttgctt agcacggcac cgtacc 46
<210> 23
   <211> 87
   <212> DNA
   <213> Artificial sequence
<220>
   <223> Description of artificial sequence: synthetic primer
<400> 23
   agacggtacc agcccgggca ggcgcgccat aacttcgtat agcatacatt atacgaagtt 60
   ataatgatga tgatgttggg aaaatgg 87
<210> 24
   <211> 20
   <212> DNA
   <213> Artificial sequence
<220>
   <223> Description of artificial sequence: synthetic primer
<400> 24
   gcagggtacc gcgtcccaga ttgtcgggtc t 31
<210> 25
   <211> 20
   <212> DNA
   <213> Artificial sequence
<220>
   <223> Description of artificial sequence: synthetic primer
<400> 25
   ccccttatga cccctctgtc 20
<210> 26
   <211> 23
   <212> DNA
   <213> Artificial sequence
<220>
   <223> Description of artificial sequence: synthetic primer
<400> 26
   gcagatgatc gagtacatcc aag 23
<210> 27
   <211> 20
   <212> DNA
   <213> Artificial sequence
<220>
   <223> Description of Artificial sequence: synthetic primer
<400> 27
   atggcggaga aagaggctac 20
<210> 28
   <211> 19
   <212> DNA
   <213> Artificial sequence
<220>
   <223> Description of artificial sequence: synthetic primer
<400> 28
   tgggagccag aaactatac 19
<210> 29
   <211> 22
   <212> DNA
   <213> Artificial sequence
<220>
   <223> Description of artificial sequence: synthetic primer
<400> 29
   ttgaatagcg agacaataac cc 22
<210> 30
   <211> 21
   <212> DNA
   <213> Artificial sequence
<220>
   <223> Description of artificial sequence: synthetic primer
<400> 30
   gcacaagggt tatatgcatc g 21
<210> 31
   <211> 63
   <212> DNA
   <213> Artificial sequence
<220>
   <223> Description of artificial sequence: synthetic primer
<400> 31
   ggggacaact ttgtatagaa aagttggcgg ccgcggatcc gatcctcgat cttgcgatgt 60
   ttc 63
<210> 32
   <211> 49
   <212> DNA
   <213> Artificial sequence
<220>
   <223> Description of artificial sequence: synthetic primer
<400> 32
   ggggactgct tttttgtaca aacttgccgt agaatccatt cggctatgg 49
<210> 33
   <211> 79
   <212> DNA
   <213> Artificial sequence
<220>
   <223> Description of artificial sequence: synthetic primer
<400> 33
   ggggacaagt ttgtacaaaa aagcaggcta cgcgtaaggc gcgccaagcg gccgcgactc 60
   tttctggcat gcggagaac 79
<210> 34
   <211> 56
   <212> DNA
   <213> Artificial sequence
<220>
   <223> Description of artificial sequence: synthetic primer
<400> 34
   ggggaccact ttgtacaaga aagctgggtc ccgggttgag tggtatgggg ccatcc 56
<210> 35
   <211> 50
   <212> DNA
   <213> Artificial sequence
<220>
   <223> Description of artificial sequence: synthetic primer
<400> 35
   ggggacagct ttcttgtaca aagtgggagg tggccgaaga ataacaaaag 50
<210> 36
   <211> 67
   <212> DNA
   <213> Artificial sequence
<220>
   <223> Description of artificial sequence: synthetic primer
<400> 36
   gggacaactt tgtataataa agttgggtac cgccagggaa ttcttgccac tccgtttatc 60
   catactg 67
<210> 37
   <211> 54
   <212> DNA
   <213> Artificial sequence
<220>
   <223> Description of artificial sequence: synthetic primer
<400> 37
   ggggacaagt ttgtacaaaa aagcaggctc ccctcgaggt cgactacatg tatc 54
<210> 38
   <211> 55
   <212> DNA
   <213> Artificial sequence
<220>
   <223> Description of artificial sequence: synthetic primer
<400> 38
   ggggaccact ttgtacaaga aagctgggtt tgcaaattaa agccttcgag cgtcc 55
<210> 39
   <211> 26
   <212> DNA
   <213> Artificial sequence
<220>
   <223> Description of artificial sequence: synthetic primer
<400> 39
   gcgaattcga gctctgtaca gtgacc 26
<210> 40
   <211> 29
   <212> DNA
   <213> Artificial sequence
<220>
   <223> Description of artificial sequence: synthetic primer
<400> 40
   agaggtacca tgggttgagt ggtatgggg 29
<210> 41
   <211> 19
   <212> DNA
   <213> Artificial sequence
<220>
   <223> Description of artificial sequence: synthetic primer
<400> 41
   ccattccgct ggattgagg 19
<210> 42
   <211> 19
   <212> DNA
   <213> Artificial sequence
<220>
   <223> Description of artificial sequence: synthetic primer
<400> 42
   atacatgtag tcgacctcg 19
<210> 43
   <211> 19
   <212> DNA
   <213> Artificial sequence
<220>
   <223> Description of artificial sequence: synthetic primer
<400> 43
   ccattccgct ggattgagg 19
<210> 44
   <211> 19
   <212> DNA
   <213> Artificial sequence
<220>
   <223> Description of artificial sequence: synthetic primer
<400> 44
   cctttgactg tgtacgtgg 19
<210> 45
   <211> 80
   <212> DNA
   <213> Artificial sequence
<220>
   <223> Description of artificial sequence: synthetic primer
<400> 45
   ggggacaagt ttgtacaaaa aagcaggcta cgcgtaaggc gcgccaagcg gccgcgactc 60
   tttctggcat gcggagagac 80
<210> 46
   <211> 56
   <212> DNA
   <213> Artificial sequence
<220>
   <223> Description of artificial sequence: synthetic primer
<400> 46
   ggggaccact ttgtacaaga aagctgggtc ccgggttgag tggtatgggg ccatcc 56
<210> 47
   <211> 35
   <212> DNA
   <213> Artificial sequence
<220>
   <223> Description of artificial sequence: synthetic primer
<400> 47
   agaacgacgc gtcacagccc gaaacaatct ctttc 35
<210> 48
   <211> 39
   <212> DNA
   <213> Artificial sequence
<220>
   <223> Description of artificial sequence: synthetic primer
<400> 48
   ttttcctttt gcggccgcct tcgcagtttc actgtctgg 39
<210> 49
   <211> 33
   <212> DNA
   <213> Artificial sequence
<220>
   <223> Description of artificial sequence: synthetic primer
<400> 49
   agaacgacgc gttttgctta tcttaacgtc tgc 33
<210> 50
   <211> 39
   <212> DNA
   <213> Artificial sequence
<220>
   <223> Description of artificial sequence: synthetic primer
<400> 50
   ttttcctttt gcggccgctg cttctcatgt tccaggtcg 39

## Description

### Field of the invention

The present invention relates to an improved method for efficient and targeted integration of nucleic acids into chromosomes of cells.

### Detailed description of the invention

Different cell types are used for different industrial purposes. For example mammalian cell lines are used for antibody production; fungal cells are preferred organisms for production of polypeptides and secondary metabolites; bacterial cells are preferred for small metabolite and antibiotic production; plant cells are preferred for taste and flavor compounds. Recombinant techniques are widely employed for optimization of the productivity of cells and/or processes. This can involve a multitude of options, including, but not limited to over expression of a gene of interest, deletion or inactivation of competing pathways, changing compartmentalization of enzymes, increasing protein or metabolite secretion, increasing organelle content and the like (see for example Khetan and Hu (1999) In: Manual of Industrial Microbiology Biotechnology, Eds. Demain and Davies, pg. 717-724). To be successful with these methods it is crucial that the recombinant construct is stably maintained in the production host. This can be either as part of an episomal vector or via integration in the genome. The latter situation is the preferred solution as this is the most stable situation. Even more preferred is the integration at the predetermined, correct genomic locus. Since in several species, especially most eukaryotic organisms, integration of DNA into the genome occurs with high frequency at random, the construction of industrial production cells by recombinant DNA technology often leads to the unwanted integration of the polynucleotide resulting in genome modifications at random. Moreover, this often results in multiple integrations and thus instable situations. This uncontrolled "at random multiple integration" of a polynucleotide is a potentially dangerous process, which can lead to unwanted modification of the genome of the host, resulting in decreased productivity.

It is therefore highly desirable to be able to construct an industrial production cell line by correct genome targeting of the polynucleotide of interest with high efficiency. Furthermore, now that the sequences of complete genomes of an increasing amount of organisms are becoming available, the opportunity to construct genome-wide over expression and deletion libraries is opened. An important requirement for the efficient construction of such libraries is that the organism in question can be efficiently transformed, that the polynucleotide of interest is correctly targeted with a high frequency and that the required homology needed to direct targeted integration of a nucleic acid into the genome is relatively short.

There are several methods described to decrease the frequency of this unwanted, at random integration of polynucleotides in cells.

Eukaryotic cells have at least two separate pathways (one via homologous and one via non-homologous recombination) through which nucleic acids (in particular of course DNA) can be integrated into the host genome. The yeast *Saccharomyces cerevisiae* is an organism with a preference for homologous recombination (HR). The ratio of homologous to non-homologous recombination (HR/NHR) of this organism may vary from about 0.9 to 1. Contrary to *Saccharomyces cerevisiae,* most higher eukaryotic cells (including fungal, plant and mammalian) cells have a preference for non-homologous recombination (NHR). Among these, the HR/NHR ratio ranges between 0.0001 and 0.5. In such organisms, the targeted integration frequency is rather low. Also, the length of homologous regions flanking a polynucleotide sequence to be integrated into the genome of such organisms has to be relatively long, for example at least 2,000 base pairs for disrupting a single gene. The necessity of such flanking regions represents a heavy burden when cloning the DNA construct comprising said polynucleotide and when transforming the organism with it. Moreover, neighboring genes which lie within those flanking regions can easily be disturbed during the recombination processes following transformation, thereby causing unwanted and unexpected side-effects.

Recently, several publications describe the inhibition of the very efficient Non-Homologous End-Joining (NHEJ) pathway, the pathway responsible for random integration of polynucleotides in cells, as a method for improving the HR/NHR ratio (see for example Ninomiya et al., 2004, Proc. Natl. Acad. Sci. USA 101:12248-12253; Krappmann et al., 2006, Eukaryot. Cell. 5:212-215). It is potentially a very powerful method, resulting in very significant improvements (even up to 60-fold) of gene targeting efficiency.

However, there are still some drawbacks to this method. Firstly, it does not work for all species. For example, mammalian cells deficient in *ku70,* one of the components of the NHEJ pathway, have been isolated (Pierce et al., 2001, Genes and Development 15: 3237-3242). These mutants have a six-fold higher homology-directed repair frequency, but no increase in the efficiency of homology-directed targeted integration. Secondly, although it has a positive effect on the NHR/HR ratio in several fungal species (see for example Ninomiya *et al.,* 2004; Krappmann *et al.,* 2006), in most cases it is limited to 60-90% correct gene targeting. This is an acceptable improvement for working with one or several genes, but not for a High Throughput genome wide analysis and/or modification of gene function. In the individual cases where 100% correct transformants were obtained this involved long flanking regions, which also is not amenable for a High Throughput genome wide analysis and/or modification of gene function. Thirdly, to obtain such strains with improved HR/NHR ratios, one has to modify the recombination machinery of the host cell and this can lead to unwanted side effects (see for example Celli et al., Nat Cell Biol (2006), 8: 885-890).

The HR/NHR ratio can also be improved by over expressing components of the HR pathway. An example of this method is given by Shaked et a/. (2005, Proc Natl. Acad. Sci. USA. 102:12265-12269). They show that by over expression of yeast RAD54 the HR frequency can be improved a 100-fold. Still, this results only 1-10% correct transformants, which makes this method not amenable for a High Throughput genome wide analysis and/or modification of gene function.

Another method is the so-called bipartite gene-targeting method (Nielsen et al., 2006, 43: 54-64). This method is using two overlapping non-functional parts of a selection marker. Upon correct homologous recombination the selection marker becomes functional. They tested the method in the fungal species with the most efficient homologous recombination system, *Aspergillus nidulans,* with 24% correct gene targeting in WT cells. The method results in a 2.5-fold improvement over the standard method, but even in *Aspergillus nidulans* only 62% of the transformants obtained is correct. Also, rather long flanking regions are used to obtain correct targeting. This is an acceptable improvement for working with one or several genes, but not for a High Throughput genome wide analysis and/or modification of gene function.

Liu et al. (2001, J. Bacteriol., 183: 1765-1772) describe another method, which uses a second selection marker to enrich for transformants with targeted gene disruption in *Acremonium chrysogenum.* The method results in a 10-fold improvement over the standard method, but still only 8% of the transformants obtained is correct.

Still another method is described by Kang and Khang (US 2005/0181509). This is a variation on the method of Liu *et al.* (2001). Here they apply a negative selection marker, *i.e*. the herpes simplex virus thymidine kinase (*HSVtk*) gene, as the second selection marker. If the selection procedure would work correctly, polynucleotides that integrate at random in the genome would kill the cells as the *HSVtk* gene would convert the 5-fluoro-2'-deoxyurine in the agar plates to a toxic compound. Again, this method increases the frequencies of correct targeting in cells, but it is limited to 50% of the cells. More importantly there is a very high percentage of false positives obtained (9-100%), which makes this method unsuitable for a High Throughput genome wide analysis and/or modification of gene function. Kang and Khang (US 2005/0181509) also describe the testing of the diphtheria toxin A (*dtA*) gene. This gene has been applied in plants and mammalian cells as a second marker to increase the frequency of correct gene targeting to 1-2% (see for examples Terada et a/., 2004, Plant Cell Rep. 22:653-659; Yagi et al., 1993, Anal. Biochem. 214:77-86). However, they failed to get this marker functional in fungal species.

Surprisingly, we found that by combining a method that increases the HR/NHR ratio with the use of a second selectable marker, we can get correct gene targeting at 100% in cells which normally have a preference for NHR. Moreover, we surprisingly found that the diphtheria toxin A (*dtA*) gene does work in filamentous fungal cells and can be used efficiently in fungal species as a second (and lethal) marker to enrich for cells wherein a correct gene targeting event has occurred.

The present invention discloses an in-vitro method to construct eukaryotic cells having a target sequence in a chromosomal DNA sequence replaced by a desired replacement sequence comprising:
modifying a parent eukaryotic cell with a preference for NHR to provide a eukaryotic cell having an increased HR/NHR ratio as compared to the parent cell,
providing a DNA molecule comprising a first DNA fragment comprising a desired replacement sequence flanked at its 5' and 3' sides by DNA sequences substantially homologous to sequences of the chromosomal DNA flanking the target sequence and a second DNA fragment comprising an expression cassette comprising a gene encoding a selection marker and regulatory sequences functional in the eukaryotic cell operably linked thereto;
transforming the modified eukaryotic cells with the DNA molecule;
growing the cells to obtain transformed progeny cells having the DNA molecule inserted into the chromosome,
deselecting the progeny cells in which the DNA molecule is inserted in the chromosome via a non-homologous recombination event by expression of the selection marker; and
obtaining cells wherein the target sequence in the chromosomal DNA sequence is replaced by the desired replacement sequence.

The parent eukaryotic cell with a preference for NHR may be any eukaryotic cell having a HR/NHR ratio ≤ 0.5, preferably ≤ 0.2, more preferably ≤ 0.1, most preferably ≤ 0.05. The exact ratio may vary between different loci in one species. Suitable loci for determining the ratio's above are the *niaD* locus (and its homologues in all species) and the *KU70* locus (and its homologues in all species).

The HR/NHR ratio is determined by analysis of a set of transformants to determine which part of the transformants has correct gene targeting (HR) and which part of the transformants has incorrect recombination (NHR). Several methods are available to obtain these data:
(i) phenotypic analysis; if the homologous target locus has an easily detectable phenotype, like the *niaD* locus (if deleted the cells become resistant to chlorate), all transformants may be tested on suitable discriminating media, for instance with and without chlorate, to obtain the frequency of HR and NHR events
(ii) PCR analysis; DNA isolated from transformants may be subjected to PCR with one primer annealing outside the flanking sequences and one primer annealing within the replacement sequence. If amplification of the expected size is obtained, this confirms a HR event. If no fragment or a fragment of the wrong size is obtained, a control PCR may be done with both primers annealing to the target locus to be replaced. If amplification of the expected size is obtained, this confirms NHR events.
(iii) Southern analysis; DNA isolated from transformants is digested with restriction enzymes that generate different hybridization patterns dependent on a HR or NHR event, or on the wild type situation.

The preferred method for quickly scanning many transformants is PCR.

Eukaryotic host cells having an increased HR/NHR ratio as compared to a parent cell may be obtainable by modifying the parent eukaryotic cell by increasing the efficiency of the HR pathway and/or by decreasing the efficiency of the NHR pathway. The HR/NHR ratio thereby is increased at least 2 times, preferably at least 4 times, more preferably at least 10 times.

In the context of the invention, the HR pathway is defined as those genes and elements being involved in the control of the targeted integration of polynucleotides into the genome of a host, said polynucleotides having a certain homology with a certain predetermined site of the genome of a host to where the integration is targeted. The NHR pathway is defined as all genes and elements being involved in the control of the integration of polynucleotides into the genome of a host irrespective of the degree of homology of the polynucleotides with a genomic sequence of the host.

Increasing the efficiency of the HR pathway and/or decreasing the efficiency of the NHR pathway may be effectuated by up and/or down regulating the expression of genes involved in HR and/or NHR. The expression level of a DNA sequence, e.g. a gene, is up or down regulated, respectively, when the expression level of this DNA sequence in the obtained eukaryotic cell is higher or lower, respectively, than the expression level of the same DNA sequence in the parent eukaryotic cell, preferably at least 2 times higher or lower, more preferably at least 4 times higher or lower, even more preferably at least 10 times higher or lower. When down regulated, the expression of the DNA sequence most preferably is not detectable.

The up and/or down regulation of the expression level of a DNA sequence may be monitored by quantifying the amount of corresponding mRNA present in a cell by Northern blotting (see for instance Molecular Cloning: A Laboratory Manual, Sambrook *et* a/., New York: Cold Spring Harbour Press, 1989) and/or by quantifying the amount of corresponding protein present in a cell by Western blotting. mRNA and protein levels may also be measured by 'omics' techniques like transcriptomics and/or proteomics. The difference in mRNA amount may also be quantified by DNA array analysis (Eisen, M. B. and Brown, P. O. DNA arrays for analysis of gene expression. Methods Enzymol. 1999: 303: 179- 205). The up and/or down regulation of the expression level of a DNA sequence may be obtained by subjecting the parent eukaryotic cell to recombinant genetic manipulation techniques and/or classical mutagenesis techniques. Up regulation of the expression of a DNA sequence using recombinant genetic manipulation techniques preferably comprises overexpression of the DNA sequence. Down regulation of the expression of a DNA sequence using recombinant genetic manipulation techniques preferably comprises inactivation of the DNA sequence. Inactivation may be done by replacing the DNA sequence by a non-functional variant thereof or by deleting part or all of the DNA sequence from the genome.

In one embodiment, the up and/or down regulation of the expression level of a DNA sequence may be inducible. This can be achieved by replacing the endogenous regulatory regions of the DNA sequence, *i.e*. a gene encoding the component involved in HR and/or NHR, by new regulatory regions, preferably comprising a repressible or inducible promoter, more preferably comprising a promoter that can be switched on/off, for instance by glucose repression, ammonia repression, and/or pH repression. Examples of fungal glucose-repressed promoters are the *Penicillium chrysogenum* pcbAB promoter (Martin et al., 1999, Antonie Van Leeuwenhoek. 75:21-31) or the *Aspergillus niger* glucoamylase promoter. Examples of on/off switchable promoters are described in the following publications: An activator/repressor dual system allows tight tetracycline-regulated gene expression in budding yeast (Belli et al., 1998, Nucl. Acid Res. 26: 942-947); a light-switch able gene promoter system (Shimizu et al., 2002, Nat. Biotech. 20:1041-1044).

A suitable eukaryotic cell may subsequently be selected by monitoring the expression level of the relevant DNA sequence. Optionally, the eukaryotic cell may be selected by measuring the efficiency of the NHR and/or of the HR pathways and/or the HR/NHR ratio. In the context of the invention, the efficiency of the HR pathway of a filamentous fungus may be measured by the efficiency of the targeted integration of a given polynucleotide sequence into a predetermined site in the genome of the filamentous fungus using given homology region(s). In the context of the invention, the efficiency of the NHR pathway of a filamentous fungus may be measured by the efficiency of the non-targeted integration of a given polynucleotide sequence in the genome of the filamentous fungus irrespective of any homology region(s).

In a preferred embodiment, eukaryotic cells having an increased HR/NHR ratio as compared to a parent cell are obtainable by decreasing the efficiency of the NHR pathway. This can be achieved by down regulating the expression of a gene involved in NHR in the eukaryotic cell as comparison to the expression of said gene in the parent eukaryotic cell measured under identical conditions.

According to a preferred embodiment, the eukaryotic cell having an increased HR/NHR ratio as compared to a parent cell is deficient in at least one of its endogenous genes that are equivalents of the following yeast genes involved in the NHR pathway: *KU70, KU80, RAD50, MRE11, XRS2, LIG4, LIF1, NEJ1* and *SIR4* (van den Bosch et al., 2002, Biol. Chem. 383: 873-892 and Allen et al., 2003, Mol. Cancer Res. 1:913-920).

An example of equivalents of the yeast *KU70* and *KU80* genes, respectively, are the genes *hdfA* or *mus51* and *hdfB* or *mus52,* respectively, from filamentous fungi (see WO05095624 and Ninomiya *et al.,* 2004). Mammalian equivalents of the yeast genes are also known as *Ku70* or *Ku80* genes (see for example Pierce *et al*., 2001).

The eukaryotic cell having an increased HR/NHR ratio as compared to a parent cell as specified hereinabove is preferably used as a host cell for transformation using the DNA molecule as specified herein below.

The first DNA fragment of the DNA molecule comprises a desired replacement sequence flanked at its 5' and 3' sides by DNA sequences substantially homologous to sequences in the chromosomal DNA flanking the target sequence.

With the term "substantially homologous" as used in this invention is meant that a DNA sequence flanking the replacement sequence has a degree of identity to a chromosomal DNA sequence flanking the target sequence of at least 80%, preferably at least 90%, over a region of not more than 3 kb, preferably not more than 2 kb, more preferably not more than 1 kb, even more preferably not more than 0.5 kb, even more preferably not more than 0.2 kb. even more preferably not more than 0.1 kb, even more preferably not more than 0.05 kb, most preferably not more than 0.03 kb. The degree of required identity may thereby depend on the length of the substantially homologous sequence. The shorter the homologous sequence, the higher the percentage homology may be.

It will be obvious to the skilled person that, in order to achieve homologous recombination via a double cross-over event, these flanking sequences need to be present at both sides of the replacement sequence and need to be substantially homologous to sequences at both sides of the target sequence in the chromosome.

The nature of the replacement sequence may vary depending on the intended use. The replacement sequence may for instance confer a selectable phenotype to the eukaryotic cell. In that case, the replacement sequence comprises a selection marker. Preferably, the selection marker is a positive selection marker. A preferred positive selection marker is the *amdS* gene. A selection marker as replacement sequence preferably is used when the target sequence needs to be inactivated.

The replacement sequence may also be a modified version of the target sequence, for instance to provide for altered regulation of a sequence of interest or expression of a modified gene product with altered properties as compared to the original gene product.

The replacement sequence may also constitute additional copies of a sequence of interest being present in the genome of a eukaryotic cell, to obtain amplification of that sequence of interest.

The replacement sequence may be a sequence homologous or heterologous to the eukaryotic cell of interest. It may be obtainable from any suitable source or may be prepared by custom synthesis.

The target sequence may be any sequence of interest. For instance, the target sequence may be a sequence of which the function is to be investigated by inactivating or modifying the sequence. The target sequence may also be a sequence of which inactivation, modification or overexpression is desirable to confer a eukaryotic cell with a desired phenotype.

The second DNA fragment comprises an expression cassette providing for expression of a selection marker. However, only a non-homologous recombination event will lead to actual integration of the selection marker cassette. This implicates that expression of this selection marker product will occur upon integration of the DNA molecule comprising the first and second DNA fragment into the chromosome of the eukaryotic cell via non-homologous recombination. Integration of the expression cassette thus will occur when the DNA molecule in integrated at a site that is not homologous to the target sequence.

Cells that express the selection marker are deselected, i.e. are removed from the population of transformed progeny cells.

In the context of the invention, the second DNA fragment encoding a selection marker confers a detectable phenotype to the recipient eukaryotic cell. Examples of detectable phenotypic selection markers are Green Fluorescent Protein (eGFP), Yellow Fluorescent Protein (eYFP), Cyan Fluorescent Protein (eCFP), Blue Fluorescent Protein (eBFP), Red Fluorescent Protein (RFP), β-galactosidase (β-gal), glucoronidase (GUS). Preferably, such detectable phenotypic selection markers are selectable markers. Examples of selectable markers are, but not limited to, tn5-ble (phleomycin^{R}), hyg (hygromycin^{R}), kan (kanamycin^{R}), gen (G418^{R}), *amdS* (acetamide utilisation).

In a preferred embodiment, the second DNA fragment encoding a detectable phenotypic selection marker is a lethal selection marker, which confers a lethal phenotype to the eukaryotic cell. Such a lethal phenotype may be achieved in various ways. A lethal phenotype for instance may directly be achieved when the lethal selection marker encodes a compound that is toxic to the cell, e.g. diphtheria toxin A or suppressor tRNA's. A lethal phenotype may also conditionally be achieved when the lethal selection marker encodes an enzyme that converts a particular substrate into a toxic compound. Examples of such conditional lethal markers, in combination with a substrate that can be converted into a toxic compound, are: nitrate reductase (*niaD* gene) in combination with chlorate, orotidine-5'-monophosphate decarboxylase (*pyrG* gene) in combination with fluoro-orotic acid, acetamidase (*amdS* gene) in combination with fluoro-acetamide, or or thymidine kinase (*tk* gene) in combination with 5-fluoro-2'-deoxyuridine.

The expression cassette providing for expression of the selection marker comprises regulatory sequences operably linked to the selection marker-encoding gene The term "operably linked" refers to a juxtaposition wherein the components described are in a relationship permitting them to function in their intended manner. A regulatory sequence such as a promoter, an enhancer or another expression regulatory signal "operably linked" to a coding sequence is positioned in such a way that expression of a polypeptide from its coding sequence is achieved under conditions compatible with the regulatory sequences.

The regulatory sequences of the selection marker expression cassette preferably are heterologous to the chromosome of the eukaryotic cell of interest, *i.e*. the regulatory sequences are from a different eukaryotic species than the eukaryotic cell of interest to be transformed. The use of a homologous regulatory sequence in this context may result in a targeted integration event at a chromosomal site corresponding to the homologous regulatory sequence. Such an integration event is undesirable because it decreases the percentage correct targeting to the site comprising the targeting sequence.

Regulatory sequences used may drive constitutive expression; this will enable to use the negative selection directly after transfection. Alternatively, regulatory sequences may be used that drive regulatable or inducible expression of the selection marker gene; this allows for a two step procedure. Firstly, the transfection and subsequent isolation of transformants is perfomed, under conditions that the transcription of the selection marker gene does not occur. Secondly, the isolated transformants are transferred to conditions which induce the transcription of the negative selection marker gene, thereby selectively killing all the isolates that underwent random integration events.

The DNA molecule may comprise the first and second DNA fragment in any order and preferably is linear. If the replacement sequence does not comprise a selection marker, such a marker may be provided on a separate DNA molecule.

A eukaryotic cell of interest is transformed with the DNA molecule comprising the first and second DNA fragment, and, optionally, a DNA molecule comprising a selection marker, using techniques commonly known in the art. Briefly, eukaryotic cells are transformed by contacting the eukaryotic cells with a suitable amount of the DNA molecule(s), preferably in linear form, and selecting colonies of transformed cells by culturing the cells on a selective medium enabling growth of transformed cells only.

Upon transformation, the DNA molecule comprising the first and second DNA fragment integrates in the chromosome of the eukaryotic host cell by a homologous or a non-homologous integration event. A homologous integration event occurs at the target sequence in the host chromosome by a double cross-over event at the homologous sequences flanking the replacement and targeting sequence. Such an event ensures that the second DNA fragment comprising the selection marker expression cassette is not integrated into the chromosome. A non-homologous integration event results in integration of the complete DNA molecule comprising first and second DNA fragments. Cells wherein a non-homologous integration event has occurred are deselected when the selection marker gene is expressed upon integration. This expression of the selection marker gene may occur simultaneously with selection of the transformants or may occur in a later stage after transformants have been selected. In the latter case, expression of the selection marker gene may be induced by a suitable inducer.

The eukaryotic cell is a eukaryotic cell with a preference for NHR. Preferably, the eukaryotic cell is a fungal, plant or animal cell. More preferably, the fungus is of the genus *Aspergillus, Penicillium, Acremonium, Trichoderma, Chrysosporium, Mortierella, Kluyveromyces* or *Pichia;* most preferably of the species *Aspergillus niger, Aspergillus nidulans, Aspergillus oryzae, Aspergillus terreus, Penicillium chrysogenum, Penicillium citrinum, Acremonium chrysogenum, Trichoderma reesei, Mortierella alpina, Chrysosporium lucknowense, Kluyveromyces lactis, Pichia pastoris* or *Pichia ciferrii.* More preferably, the plant is of the genus *Arabidopsis, Nicotiana, Solanum, Lactuca, Brassica, Oryza, Asparagus, Pisum, Medicago, Zea, Hordeum, Secale, Triticum, Capsicum, Cucumis, Cucurbita, Citrullis, Citrus, Sorghum;* most preferably of the species *Arabidopsis thaliana, Nicotiana tabaccum, Solanum lycopersicum, Solanum tuberosum, Solanum melongena, Solanum esculentum, Lactuca sativa, Brassica napus, Brassica oleracea, Brassica rapa, Oryza glaberrima, Oryza sativa, Asparagus officinalis, Pisum sativum, Medicago sativa, Zea mays, Hordeum vulgare, Secale cereale, Triticum aestivum, Triticum durum, Capsicum sativus, Cucurbita pepo, Citrullus lanatus, Cucumis melo, Citrus aurantifolia, Citrus maxima, Citrus medica, Citrus reticulata.* More preferably; the animal cell is of the genus *Homo, Rattus, Mus, Sus, Bos, Danio, Canis, Felis, Equus, Salmo, Oncorhynchus, Gallus, Meleagris, Drosophila, Caenorhabditis;* most preferably of the species *Homo sapies, Rattus norvegicus, Mus musculus, Sus scrofa, Bos taurus, Danio rerio, Canis lupus, Felis catus, Equus caballus, Salmo salar, Oncorhynchus mykiss, Gallus gallus, Meleagris gallopavo, Drosophila melanogaster, Caenorhabditis elegans.*

By using eukaryotic cells with a increased HR/NHR ratio, the method of the invention advantageously ensures an increased targeting of a polynucleotide to a chromosomal target site of interest. The method of the invention further advantageously allows the provision of transformed eukaryotic cells that are enriched in cells wherein the correct targeted integration event has occurred. In particular, at least 50% of the transformed clones has the replacement sequence targeted to the target sequence in the chromosome, as compared to 1-2% in a transformation with a targeting construct without a second fragment comprising a selection marker.

### Description of the Figures

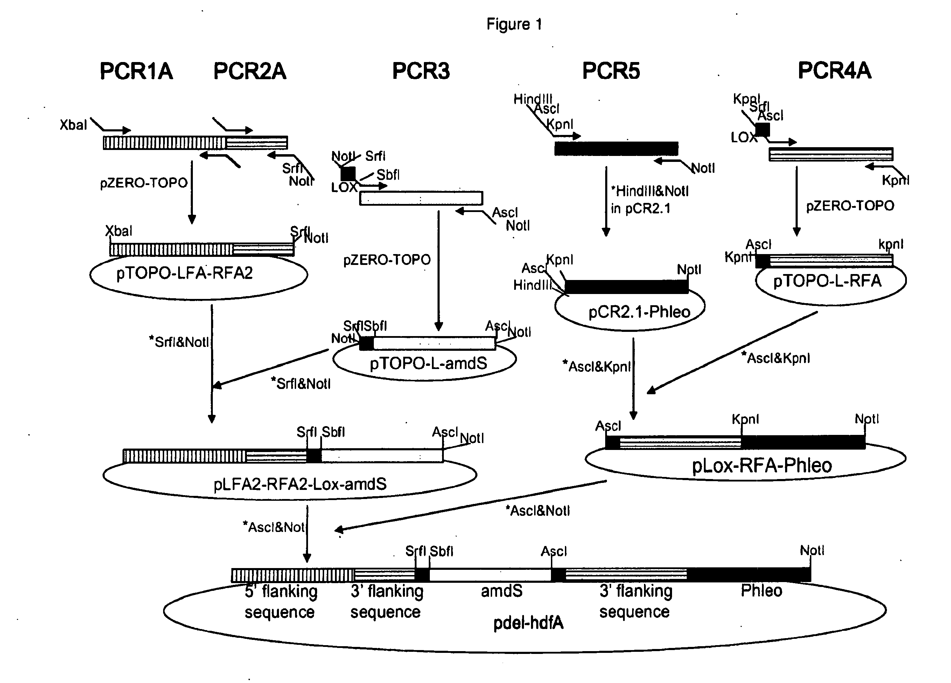

Figure 1 shows the cloning scheme of plasmid pdel-hdfA. The details of the five PCR reactions PCR1 A, PCR2A, PCR3, PCR4A and PCR5 are specified in example 1. Each intermediate plasmid name and relevant restriction enzyme sites are indicated. Legend: * = illustrates restriction digest with enzymes; boxes with vertical lines = 5' homologous flanking sequence; boxes with horizontal lines = 3' homologous fl; anking sequence; white boxes = *amdS* selection marker cassette; large black boxes = phleomycin selection marker cassettes; small black boxes = recombination sequences

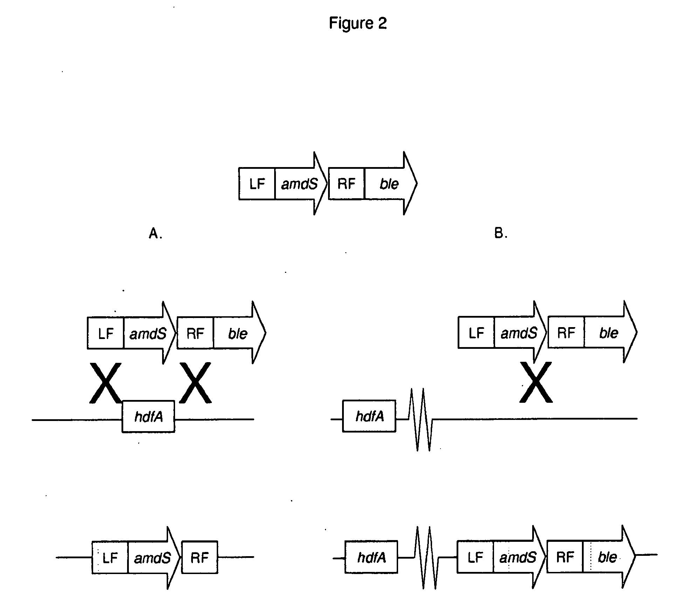

Figure 2 shows the two different recombination pathways in fungi. A. (Double) Homologous Recombination, resulting in a targeted integration event. In this case the non-homologous *ble* gene would not integrate, thereby rendering the transformant sensitive to phleomycin. B. Non-Homologous Recombination, resulting in a random integration event. In this case the non-homologous *ble* gene will integrate, thereby rendering the transformant resistant to phleomycin. On top of this the target locus, in this case *hdfA,* stays intact. Legend: LF = 5' homologous flanking sequence; *amdS = amdS* selection marker cassette; RF = 3'homologous flanking sequence; ble = phleomycin selection marker cassette; hdfA = Penicillium chrysogenum hdfA gene (yeast KU70 homologue). The 'X' represent recombination events.

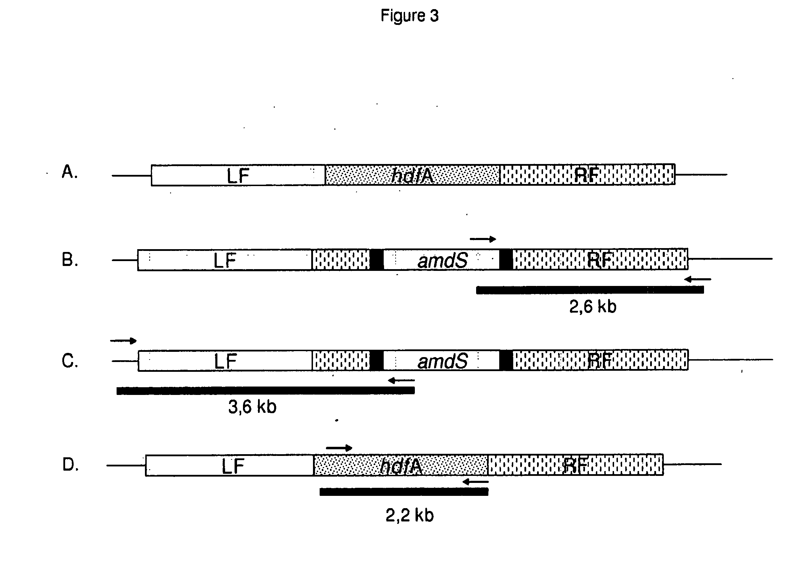

Figure 3 shows the PCR controls to determine if the *Penicillium chrysogenum hdfA* gene was deleted correctly. A. The WT locus. B. Correct gene targeting would produce a 2.6 kb fragment with oligonucleotide SEQ ID NO 11 and SEQ ID NO 12; the WT should not produce a fragment. C. Correct gene targeting would produce a 3.6 kb fragment with oligonucleotide SEQ ID NO 13 and SEQ ID NO 14; the WT should not produce a fragment. D. Correct gene targeting would not produce a fragment with oligonucleotide SEQ ID NO 15 and SEQ ID NO 16; the WT should produce a 2.2 kb fragment. Legend: LF = 5' homologous flanking sequence; *amdS = amdS* selection marker cassette; RF = 3' homologous flanking sequence; hdfA = *Penicillium chrysogenum* hdfA gene (yeast KU70 homologue). The arrows indicate the annealing position of the oligonucleotides as specified in the text of example 1. The thick lines indicate the PCR fragment amplified in the PCR reactions as specified in the text of example 1.

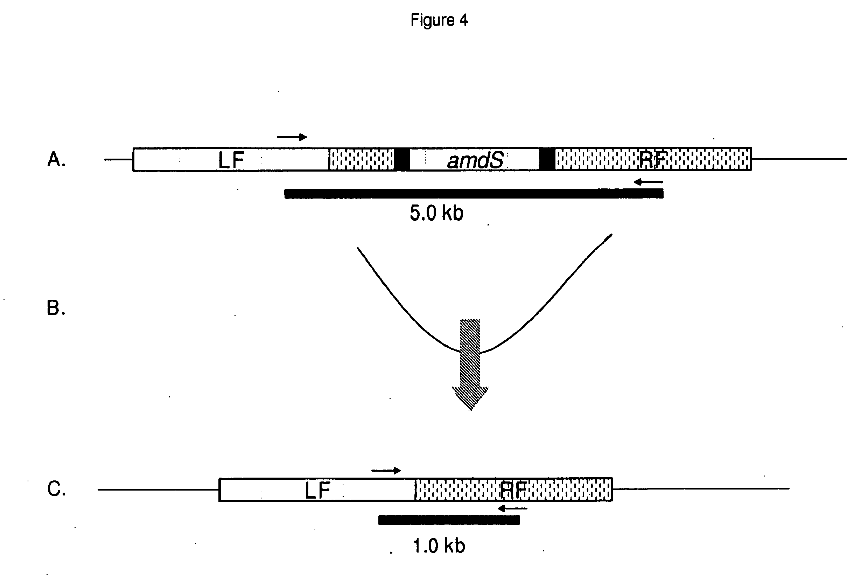

Figure 4 is the schematic representation of the PCR controls to determine if the marker-free version of the *Penicillium chrysogenum hdfA* deletion mutant was obtained. *A. hdfA* deletion with the *amdS* marker still present should produce a 5 kb fragment with oligonucleotide SEQ ID NO 17 and SEQ ID NO 18. B. Schematic representation of the recombination event selected, enabling the cells to become resistant versus fluoroacetamide. B. Marker-free *hdfA* deletion should produce a 1 kb fragment with oligonucleotide SEQ ID NO 17 and SEQ ID NO 18. Legend: LF = 5' homologous flanking sequence; *amdS = amdS* selection marker cassette; RF = 3'homologous flanking sequence; The arrows indicate the annealing position of the oligonucleotides as specified in the text of example 1. The thick lines indicate the PCR fragment amplified in the PCR reactions as specified in the text of example 1.

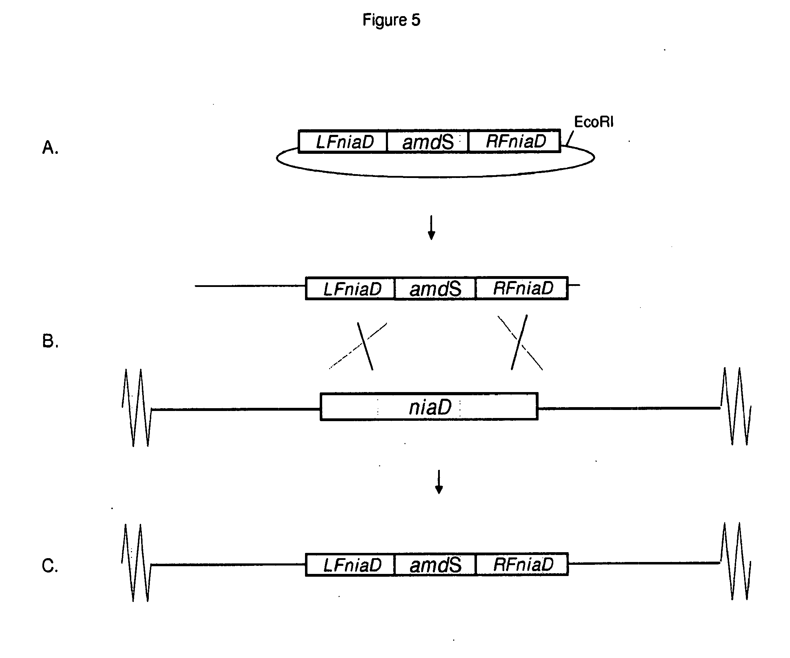

Figure 5 shows the approach for targeted integration at the niaD locus of Penicillium chrysogenum. A. The plasmid pDESTR4R3N*amdS*N. B. The linearized pDESTR4R3N*amdS*N induced double homologous recombination (or cross-over) at the genomic niaD locus. C. The genomic niaD locus organization after *amdS* exchange. Legend: LFniaD = 5' homologous flanking sequence of the niaD gene locus; *amdS = amdS* selection marker cassette; RFniaD = 3' homologous flanking sequence of the niaD gene locus; The 'X' represent recombination events.

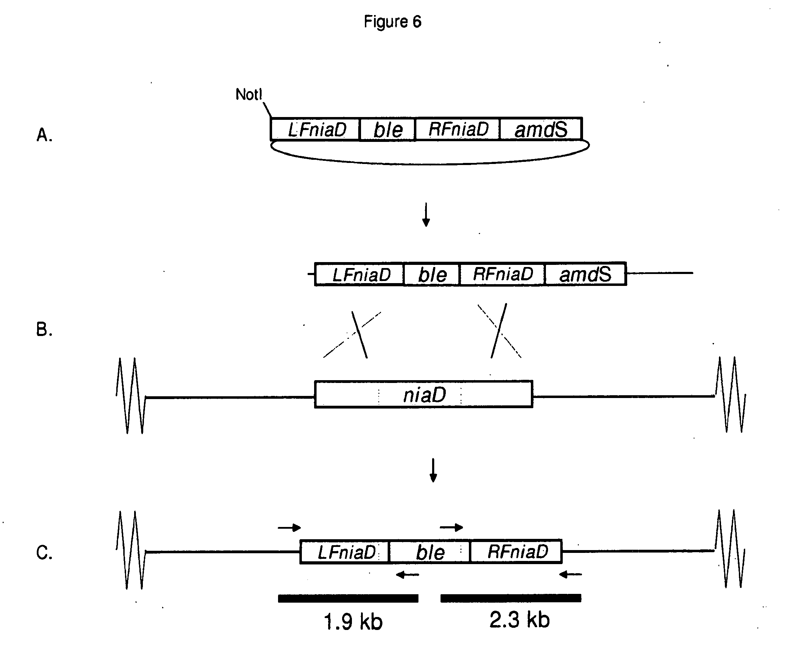

Figure 6 shows the approach for targeted integration at the niaD locus of Penicillium chrysogenum by using a second selectable marker in combination with strains which have an improved frequency of homologous recombination. A. The plasmid pDESTR4R3NphleoN*amdS*. B. The linearized pDESTR4R3NphleoN*amdS* induced double homologous recombination (or cross-over) at the genomic niaD locus. C. The genomic niaD locus organization after ble exchange and loss of the *amdS* gene. The PCR amplification to verify correct gene targeting are indicated, including the expected fragment sizes. Legend: LFniaD = 5' homologous flanking sequence of the niaD gene locus; ble = phleomycin selection marker cassette; RFniaD = 3' homologous flanking sequence of the niaD gene locus; *amdS* = *amdS* selection marker cassette; The 'X' represent recombination events. The arrows indicate the annealing position of the oligonucleotides as specified in the text of example 4. The thick lines indicate the PCR fragment amplified in the PCR reactions as specified in the text of example 4.

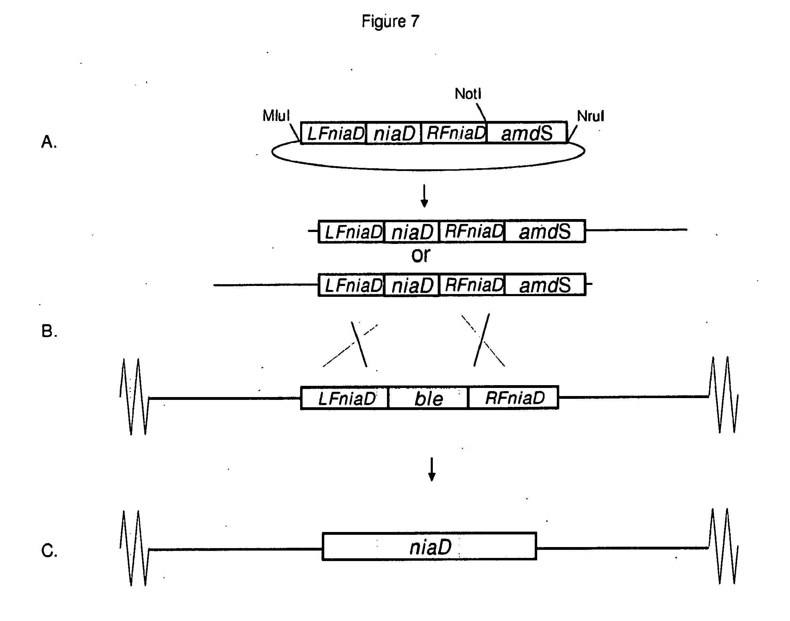

Figure 7 shows the approach for targeted restoration of the niaD locus of Penicillium chrysogenum by using a second selectable marker in combination with strains which have an improved frequency of homologous recombination. A. Schematic representation of the plasmids pDONR201 *amdS*hi800 and pDONR201 *amdS*ni1200. B. The linearized vectors pDONR201 *amdS*ni800 and pDONR201 *amdS*ni1200 (either via Mlul or Nrul) impose double homologous recombination (or cross-over) at the genomic niaD locus. C. The genomic niaD locus organization after restoration of the central 441 bp. Legend: LFniaD = 5' homologous flanking sequence of the niaD gene locus; niaD = partial niaD open reading frame; RFniaD = 3' homologous flanking sequence of the niaD gene locus; *amdS* = *amdS* selection marker cassette; The 'X' represent recombination events. The arrows indicate the annealing position of the oligonucleotides as specified in the text of example 5. The thick lines indicate the PCR fragment amplified in the PCR reactions as specified in the text of example 5.

### Examples

### General materials and methods

Standard procedures were carried out as described elsewhere (Sambrook et al., 1989, Molecular cloning: a laboratory manual, 2nd Ed., Cold Spring Harbor Laboratory Press, Cold Spring Harbor, New York). DNA for plasmid construction was amplified using a proofreading polymerase, Phusion (Finnzymes) following the manufacturer's protocol; while verification of constructed strains and plasmids was achieved by using Taq polymerase. Restriction enzymes were from Invitrogen or New England Biolabs. For routine cloning, *Escherichia coli* strains Top10 and DH10B (Invitrogen) were employed. The Gateway system of Invitrogen was applied according to the manufacturer's manuals. Verification of the constructed plasmids was carried out by restriction analysis and subsequent sequencing.

### Example 1

### Deletion of the hdfA gene of Penicillium chrysogenum

### Vector construction

To delete the chromosomal copy of the *Penicillium chrysogenum hdfA* gene (see WO05095624), the fungal equivalent of the yeast KU70 gene involved in the Non-Homologous End-Joining pathway, the integration vector pdel-*hdfA* was constructed. To this end 2500 bp genomic DNA fragments both directly upstream and directly downstream of the *hdfA* gene were PCR amplified. The upstream region (alias left flanking; PCR1A in Fig. 1) was PCR amplified from genomic DNA using the oligonucleotides of SEQ ID NO 1 and SEQ ID NO 2, thereby introducing an Xbal site at the far left end. To facilitate later removal of the *amdS* selection marker a 1000 bp repeat surrounding this cassette was introduced in the construct, by PCR amplification of the first 1000 bp downstream of the *hdfA* gene from genomic DNA and fusing this to the left flanking (PCR2A in Fig. 1). For this the oligonucleotides of SEQ ID NO 3 and SEQ ID NO 4 where used, introducing *Srf*l and *Not*l sites at the right end. The whole fragment (3.5 kb) was cloned in pZERO-TOPO (invitrogen), yielding plasmid pTOPO-LFA-RFA2. The *amdS* selection marker cassette was PCR amplified from pHELY-A1 (see WO04106347) using oligonucleotides of SEQ ID NO 5 and SEQ ID NO 6, introducing *Notl, Srf*l and *Sbfl* sites on the left end and Ascl and *Notl* sites on the right end (PCR3 in Fig. 1). Also this fragment was cloned in to pZERO-TOPO (*i.e*. yielding plasmid pTOPO-L-amdS) and subsequently the *Srf*I*-Not*I fragment was recloned into pTOPO-LFA-RFA2, yielding plasmid pLFA2-RFA2-Lox-amdS. The downstream region (alias right flanking; PCR4A in Fig. 1) was PCR amplified from genomic DNA using the oligonucleotides of SEQ ID NO 7 and SEQ ID NO 8, thereby introducing Kpnl, Srfl and AscI sites at the left end and a Kpnl site on the right end. Again the fragment was cloned in to pZERO-TOPO, yielding plasmid pTOPO-L-RFA. To facilitate later an efficient screening for targeted integration at the *hdfA* locus, the phleomycin resistance cassette was PCR amplified (PCR5 in Fig. 1; Phleo) from pAMPF21 (Fierro et al., 1996, Curr Genet 29: 482-489) using the oligonucleotides of SEQ ID NO 9 and SEQ ID NO 10, thereby introducing *Hin*dIII*, Asc*l and *Kpn*I sites at the left end and *Not*I*,* at the right end. The fragment was digested with *Hind*III and *Not*l and cloned in to pCR2.1, yielding pCR2.1-Phleo. The right flanking was isolated from pTOPO-L-RFA after digestion with Ascl and *Kpn*I*;* and cloned into pCR2.1-Phleo, yielding plasmid pLox-RFA-Phleo. The complete deletion construct of *hdfA* was obtained after isolating the *Acsl-Notl* fragment of pLox-RFA-Phleo and ligating this in pLFA-RFA2-Lox-*amdS* digested with *Acs*I*-Not*I*,* yielding plasmid pdel*-hdfA* (see Fig.1).

### Deletion of chromosomal copy

The deletion fragment was isolated from plasmid pdel*-hdfA* as a *Sfi*l fragment and transfected to *Penicillium chrysogenum* protoplasts. *Penicillium chrysogenum* protoplasts were produced according to standard protocols (see for examples Cantoral et al., 1987, Biotechnology 5: 494-497; Swinkels et al., 1997, WO97/06261) however Glucanex^{™} (Sigma) was applied as lysing enzyme. After transfection the protoplasts were plated out on selective regeneration agar plates with acetamide as the sole nitrogen source (for an exact description of the media, see Swinkels *et al.,* 1997). Acetamide utilizing colonies were transferred to fresh acetamide plates (without saccharose to induce sporulation) and 300 of these were subsequently screened for phleomycin sensitivity (on mineral medium agar as described for *Penicillium chrysogenum* in US 2002/0039758, without phenyl acetic acid but supplemented with 15 g/L agar to solidify and 50 mg/L phleomycin). The thus obtained transformants should be enriched for targeted integration at the *hdfA* locus as the random transformants would be phleomycin resistant (see Fig. 2). Out of the 300 acetamide utilizing transformants only 9 were phleomycin sensitive. This suggested that 3% of the original transformants were putative correct transformants, *i.e*. targeting towards the *hdfA* could be successful. This is in line with values reported for filamentous fungi were in WT situations most of the transformants are the result of random integration. By adding the screening for phleomycin sensitivity we could efficiently deselect these and put more effort on a limited number (*i.e*. 9) of putative *hdfA* mutants.

### Confirmation of hdfA deletion

Six of the 9 putative *hdfA* mutants were screened with colony PCR to determine if the deletion was correct. To this end a piece of the sporulated colony was resuspended in 50 µl of water and boiled for 10 minutes at 98 degrees Celsius. The cell debris was spun down and one µl of the supernatant was used in 3 PCR reactions (see Fig. 3). Using a primer set with the forward primer annealing at the terminator of the *amdS* cassette and the reverse primer annealing downstream of the right flank used in the deletion cassette only transformants with the correct integration event should give a 2.6 kb fragment (see Fig.3B). The PCR reaction with oligonucleotides SEQ ID NO 11 and SEQ ID NO 12 showed that all 6 candidates had the correct integration at this site. Using a primer set with the forward primer annealing upstream of the left flank used in the deletion cassette and the reverse primer annealing at the *gpdA* promoter of the *amdS* cassette only transformants with the correct integration event should give a 3.6 kb fragment (See Fig. 3C). The PCR reaction with oligonucleotides SEQ ID NO 13 and SEQ ID NO 14 showed that only one of the candidates had the correct integration at this site. Using a primer set with the forward primer starting at the ATG of the *hdfA* gene and the reverse primer starting just in front of the STOP codon of the *hdfA* transformants with the correct integration event should not produce a fragment. The PCR reaction with oligonucleotides SEQ ID NO 15 and SEQ ID NO 16 showed that single candidate left after the first two PCRS indeed gave no band, while in the other five candidates *hdfA* could still be amplified, indicating that in this latter group an odd recombination took place at the *hdfA* locus. However, strain SA1 was confirmed to be a real *hdfA* deletion strain. So in conclusion 1 out of 6 transformants has a correct *hdfA* gene replacement.

### Obtaining a marker-free hdfA mutant

In order to obtain a clean *hdfA* mutant the *amdS* selection marker cassette had to be removed. To facilitate this, a 1000 bp repeat was introduced during cloning (see Fig.1). This 1000 bp is exactly the same as the first 1000 bp of the right flanking region used in the deletion construct. Thus, if a recombination would take place at this 1000 bp a clean *hdfA* delection should be obtained in which the flanking regions are retained and the complete *hdfA* locus will be removed. The *amdS* selection marker has the advantage that one can select for the presence (*i.e*. enabling growth on acetamide as sole nitrogen source) as well on the absence (*i.e*. fluoroacetamide would generate toxic fluorine in the presence of *amdS*) of the gene. Spores of strains SA1 were plated on fluoroacetamide containing agar plates (WO9706261) and growing colonies were isolated and checked with colony PCR. To this end PCR template material was obtained as described above and the PCR was run using the oligonucleotides SEQ ID NO 17 and SEQ ID NO 18. In case of SA1 this should give a fragment of 5 kb, while in the recombined situation *(i.e.* marker-free *hdfA* deletion) this should give a fragment of 1 kb (see Fig. 4). Ten fluoroacetamide resistant colonies were checked and all showed the 1 kb band. On top of this, none of these 10 candidates was able to grow on acetamide plates, confirming that double homologous recombination has taken place: the *amdS* gene has been removed and these colonies are true *hdfA* marker-free deletion strains (AS1 to AS10).

### Phenotype of the marker-free hdfA mutant

To determine if the *hdfA* mutants had no obvious phenotype 5 items were checked: penicillin V production, morphology, growth rate, sporulation and phleomycin sensitivity. Penicillin V production was checked in liquid medium as described in US 2002/0039758. Four strains were checked: the WT, SA1 (*hdfA* mutant containing *amdS*)*,* AS1 and AS2 (*hdfA* mutants both marker-free). The productivities were respectively 100%, 114%, 105% and 100%. So, the *hdfA* deletion causes no apparent change towards penicillin production. Morphology was checked by growing the mutants on plate. No significant difference could be observed versus the WT. So, the *hdfA* deletion causes no apparent change towards morphology. Growth rate was checked during the Penicillin V productivity tests as described above by measuring the OD600. No significant difference could be observed versus the WT. So, the *hdfA* deletion causes no apparent change towards growth rate. Sporulation was checked by visually comparing the coloring and structure of the colonies on agar plates. No significant difference could be observed versus the WT. So, the *hdfA* deletion causes no apparent change towards sporulation. As the *hdfA* gene product possibly has a role in repair mechanisms the sensitivity versus phleomycin, a known inducer of DNA breaks, was checked. This was done by testing the growth on mineral medium agar plates as described for *Penicillium chrysogenum* in UPS 2002/0039756, without phenyl acetic acid but supplemented with 15 g/L agar to solidify and 0-50 mg/L phleomycin. No significant difference could be observed versus the WT. So, the *hdfA* deletion causes no apparent change towards phleomycin sensitivity. Finally, we concluded that the *hdfA* mutant has no apparent phenotype and can be used for further studies.

### Example 2

### Deletion of the hdfB gene of Penicillium chrysogenum

### Vector construction

To delete the chromosomal copy of the *Penicillium chrysogenum hdfB* gene (see WO05095624), the fungal equivalent of the yeast *KU80* gene involved in the Non-Homologous End-Joining pathway, the integration vector pdel-*hdfB* was constructed. The construction was generally the same as outlined in Fig. 1 for the *hdfA* gene. First, two 2500 bp genomic DNA fragments directly upstream and directly downstream the *hdfB* gene were PCR amplified. The upstream region (alias left flanking) was PCR amplified from genomic DNA using the oligonucleotides of SEQ ID NO 19 and SEQ ID NO 20, thereby introducing an *Xba*I site at the far left end. To facilitate later removal of the *amdS* selection marker a 1000 bp repeat surrounding this cassette was introduced in the construct, by PCR amplification of the first 1000 bp downstream of the *hdfB* gene from genomic DNA and fusing this to the left flanking. For this the oligonucleotides of SEQ ID NO 21 and SEQ ID NO 22 where used, introducing *Srf*I and *Not*I sites at the right end. The whole fragment (3.5 kb) was cloned in pZERO-TOPO (Invitrogen), yielding plasmid pTOPO-LFB-RFB2. The *Srf*I*-Not*I fragment of pTOPO-L-amdS was recloned into pTOPO-LFB-RFB2, yielding plasmid pLFB2-RFB2-Lox-amdS. The downstream region (alias right flanking) of *hdfB* was PCR amplified from genomic DNA using the oligonucleotides of SEQ ID NO 23 and SEQ ID NO 24, thereby introducing *Kpn*l*, Srfl* and *Asc*l sites at the left end and a *Kpn*I site on the right end. The fragment was cloned in to pZERO-TOPO, yielding plasmid pTOPO-L-RFB. This right flanking was isolated from pTOPO-L-RFB after digestion with *Asc*I and *Kpn*I*;* and cloned into pCR2.1-Phleo, yielding plasmid pLox-RFB-Phleo. The complete deletion construct of *hdfB* was obtained after isolating the *Acs*I*-Not*I fragment of pLox-RFB-Phleo and ligating this in pLFB-RFB2-Lox-*amdS* digested with *Acs*I-*Not*l, yielding plasmid pdel-*hdfB* (see Fig.1).

### Deletion of chromosomal copy

The deletion fragment was isolated from plasmid pdel*-hdfB* as a *Sfi*I fragment and transfected to *Penicillium chrysogenum* protoplasts (produced according to standard protocols, *i.e*. Cantoral et al., 1987, Biotechnology 5: 494-497; Swinkels et al., 1997, WO97/06261) however Glucanex^{™} (Sigma) was applied as lysing enzyme. After transfection the protoplasts were plated out on selective regeneration agar plates with acetamide as the sole nitrogen source (media described in Swinkels *et al*., 1997). Acetamide utilizing colonies were transferred to fresh acetamide plates (without saccharose to induce sporulation) and 375 of these were screened for phleomycin sensitivity (on mineral medium agar as described for *Penicillium chrysogenum* in US 2002/0039758, without phenyl acetic acid but supplemented with 15 g/L agar to solidify and 50 mg/L phleomycin). The thus obtained transformants should be enriched for targeted integration at the *hdfA* locus as the random transformants would be phleomycin resistant (outlined for *hdfA* in Fig. 2). Out of the 375 acetamide utilizing transformants only 12 were phleomycin sensitive. This suggested that 3.2% of the original transformants were putative correct transformants, *i.e*. targeting towards the *hdfB* gene could be successful. This is in line with values reported for filamentous fungi were in WT situations most of the transformants are the result of random integration. By adding the screening for phleomycin sensitivity we could efficiently deselect these and put more effort on a limited number (*i.e*. 12) of putative *hdfB* mutants.

### Confirmation of hdfB deletion

Eight of the 12 putative *hdfB* mutants were screened with colony PCR to determine if the deletion was correct. To this end, part of the sporulated colony was resuspended in 50 µl of water and boiled for 10 min at 98°C. The cell debris was spun down and one µl of the supernatant was used in 3 PCR reactions (similar set-up as outlined for *hdfA* in Fig. 3). Using a primer set with the forward primer annealing at the terminator of the *amdS* cassette and the reverse primer annealing downstream of the right flank used in the deletion cassette only transformants with the correct integration event should give a 2.6 kb fragment. The PCR reaction with oligonucleotides SEQ ID NO 11 and SEQ ID NO 25 showed that all 8 candidates had the correct integration at this site. Using a primer set with the forward primer annealing upstream of the left flank used in the deletion cassette and the reverse primer annealing at the *gpdA* promoter of the *amdS* cassette only transformants with the correct integration event should give a 3.6 kb fragment. The PCR reaction with oligonucleotides SEQ ID NO 26 and SEQ ID NO 14 showed that 7 out of the 8 candidates had the correct integration at this site. Using a primer set with the forward primer starting at the ATG of the *hdfb* gene and the reverse primer starting just in front of the STOP codon of the *hdfB* transformants with the correct integration event should not produce a fragment. The PCR reaction with oligonucleotides SEQ ID NO 27 and SEQ ID NO 28 showed that from the 7 candidates left after the first two PCRs 5 indeed gave no band, while in the other three candidates (parts of) *hdfB* could still be amplified, indicating that in this latter group an odd recombination took place at the *hdfB* locus. However, strains SB2, SB3, SB4, SB5 and SB12 were confirmed to be real *hdfB* deletion strains. So in conclusion 5 out of 8 transformants have a correct *hdfB* gene replacement.

### Obtaining marker-free hdfB mutants

In order to obtain a clean *hdfB* mutant the *amdS* selection marker had to be removed. To facilitate this, a 1000 bp repeat was introduced during cloning (see above). This 1000 bp is exactly the same as the first 1000 bp of the right flanking region used in the deletion construct. Thus, if a recombination would take place at this 1000 bp a clean *hdfB* delection should be obtained in which the flanking regions are retained and the complete *hdfB* locus will be removed. The *amdS* selection marker has the advantage that one can select for the presence (*i.e*. enabling growth on acteamide as the sole nitrogen source) as well on the absence (*i.e*. fluoroacetamide would generate toxic fluor in the presence of *amdS)* of the gene. Spores of the 5 *hdfB* strains were plated on fluoroacetamide containing agar plates (see WO9706261) and growing colonies were isolated and checked with colony PCR. To this end PCR template material was obtained as described above and the PCR was run using the oligonucleotides SEQ ID NO 29 and SEQ ID NO 30. If *amdS* would be present this should give a fragment of 5.5 kb, while in the recombined situation (*i.e*. marker-free *hdfB* deletion) this should give a fragment of 1 kb (as outlined for *hdfA* in Fig. 4). Ten fluoroacetamide resistant colonies were checked for each of the 5 *hdfB* strains and all derivatives showed the 1 kb band. On top of this, none of these 50 derivatives was able to grow on acetamide plates, confirming that the recombination took place: the *amdS* gene has been removed and these are true *hdfB* marker-free deletion strains.

### Phenotype of the marker-free hdfB mutant

As for the *hdfA* mutant we determined if the *hdfB* mutants had no obvious phenotypes. The same 5 items were checked: penicillin V production, morphology, growth rate, sporulation and phleomycin sensitivity. Penicillin V production was checked in liquid medium as in US 2002/0039758. Two marker-free derivatives were checked per *amdS-*positive *hdfB* strain and compared to the productivity of the WT strain (Table 1).

**Table 1. PenicillinV productivities of hdfB mutants; Productivity of the WT is set at 100.**

| AMDS | + | | - | | | |
|---|---|---|---|---|---|---|
| | Strain | Pen V | Strain | Pen V | Strain | Pen V |
| | SB2 | 108 | BS1 | 98 | BS2 | 100 |
| | SB3 | 106 | BS3 | 100 | BS4 | 102 |
| | SB4 | 100 | BS5 | 98 | BS6 | 101 |
| | SB5 | 116 | BS7 | 101 | BS8 | 97 |
| | SB12 | 92 | BS9 | 86 | BS10 | 88 |

The productivities all are around 100 +/- 10% of the WT strains. So, the *hdfB* deletion causes no apparent change towards penicillin production. Morphology was checked by growing the mutants on plate. No significant difference could be observed versus the WT. So, the *hdfB* deletion causes no apparent change towards morphology. Growth rate was checked during the PenicillinV productivity tests as described above by measuring the OD600. No significant difference could be observed versus the WT. So, the *hdfB* deletion causes no apparent change towards growth rate. Sporulation was checked by visually comparing the coloring and structure of the colonies on agar plates. No significant difference could be observed versus the WT. So, the *hdfB* deletion causes no apparent change towards sporulation. As the *hdfB* gene product possibly has a role in repair mechanisms the sensitivity versus phleomycin, a known inducer of DNA breaks, was check. This was done by testing the growth on mineral medium agar plates as described for *Penicillium chrysogenum* in US 2002/0039758, without phenyl acetic acid but supplemented with 15 g/L agar to solidify and 0-50 mg/L phleomycin. No significant difference could be observed versus the WT. So, the *hdfB* deletion causes no apparent change towards phleomycin sensitivity. Finally, we concluded that, like the *hdfA* mutant, the *hdfB* mutant has no apparent phenotype and can be used for further studies.

### Example 3

### Homologous recombination frequencies in hdfA or hdfB mutant strains of Penicillium chrysogenum

To determine the homologous recombination frequency of *hdfA* and *hdfB* deletion strains compared to the WT a deletion cassette for the *niaD* locus, encoding nitrate reductase, was constructed. To this end the two 1800 bp fragments of the *niaD* locus were amplified, these were separated by the *amdS* expression cassette and should enable the exchange of part of the *niaD* gene for the *amdS* expression cassette, thereby rendering the transformants chlorate resistant (due to the deletion of *niaD*) and able to use acetamide as the sole nitrogen source. Three PCR reactions were performed (see Table 2) and cloned into the Gateway pDONR vectors of Invitrogen, enabling later efficient fusion via the Multi-Gateway system (see www.Invitrogen.com).

**Table 2. Oligonucleotides and pENTR vectors used for cloning of niaD-deletion cassette.**

| Fragment | Template | Forward oligonucleotide | Reverse oligonucleotide | pENTR vector |
|---|---|---|---|---|
| 5'niaD | Genomic DNA | SEQ ID NO 31 | SEQ ID NO 32 | pDONR^{™}P4-P1R |
| PgpdA-*amdS* | pHELY-A1 | SEQ ID NO 33 | SEQ ID NO 34 | pDONR^{™}221 |
| 3'niaD | Genomic DNA | SEQ ID NO 35 | SEQ ID NO 36 | pDONR^{™}P2R-P3 |

The three obtained vectors were used to perform a multi-site Gateway reaction into pDESTR4-R3, yielding plasmid pDESTR4R3*NamdS*N (see Fig. 5). This plasmid was linearized with EcoRI and transfected to *Penicillium chrysogenum* protoplasts. *Penicillium chrysogenum* protoplasts were produced according to standard protocols (see for examples Cantoral et al., 1987, Biotechnology 5: 494-497; Swinkels et al., 1997, WO97/06261) however Glucanex^{™} (Sigma) was applied as lysing enzyme. After transfection the protoplasts were plated out on selective regeneration agar plates with acetamide as the sole nitrogen source (for an exact description of the media, see Swinkels *et al.,* 1997). Acetamide utilizing colonies were transferred to fresh acetamide plates (without saccharose to induce sporulation) and 100 of these were subsequently screened for chlorate sensitivity (per liter: NaCl, 3 g; KH₂PO₄, 1 g; FeSO₄. 7 H₂O, 10 mg; MgSO₄. 7 H₂O, 0.5 g; Oxoid Agar no.1, 15 g; Glucose, 20 g; spore elements, see WO9706261 page 10-11, all components from ZnSO₄ to biotin; adenine, 1.85 g; KClO₃, 12.5 g). From the results presented in Table 3 it can be clearly concluded that the deletion of either *hdfA* or *hdfB* has a significant effect on the frequency of targeted integration: targeted integration increased from 1% in the WT to 47-56% in the mutants.

**Table 3. Gene targeting frequencies in WT, hdfA and hdfB mutants.**

| Strain | Acetamide transformants | Chlorate resistant |
|---|---|---|
| WT | +/- 1400 | 1 % |
| *hdfA* mutant SA1 | 222 | 56% |
| *hdfB mutant* SB1 | 163 | 47% |

### Example 4

### Improved homologous recombination frequencies in hdfA or hdfB mutant strains of Penicillium chrysogenum using a second selectable marker

To determine the effect of a second selectable marker the deletion construct pDESTR4R3NphleoN*amdS* (see Fig. 6) was obtained. Again, the homologous recombination frequency of *hdfA* and *hdfB* deletion strains was compared to the WT, after disruption of the *niaD* locus. To this end the same 1800 bp flanking fragments of the *niaD* locus were used as described in example 3, but now these were separated by the ble expression cassette, resulting in phleomycin resistance. Situated outside the homologous flanking regions, the *amdS* expression cassette could be used to deselect any undesired transformants due to a non-homologous integration event. In fact it would be possible to directly select for correct transformants on fluoroacetamide/phleomycin selection plates (even without chlorate). One PCR reaction was performed to amplify the *ble* gene cassette and introduce the necessary Gateway sites (see table 4); for completeness sake the two flanking region PCRs are copied from example 3.

**Table 4. Oligonucleotides and pENTR vectors used for construction of pDESTR4R3NphleoNamdS.**

| Fragment | Template | Forward oligonucleotide | Reverse oligonucleotide | pENTR vector |
|---|---|---|---|---|
| 5'niaD | Genomic DNA | SEQ ID NO 31 | SEQ ID NO 32 | pDONR™P4-P1R |
| *ble* | pAMPF21 | SEQ ID NO 37 | SEQ ID NO 38 | pDONR™221 |
| 3'niaD | Genomic DNA | SEQ ID NO 35 | SEQ ID NO 36 | pDONR™P2R-P3 |

The three obtained vectors were used to perform a multi-site Gateway reaction into pDESTR4-R3, yielding plasmid pDESTR4R3NphleoN. The P*gpdA-amdS* fragment can be isolated as a *Kpnl-Eco*RI fragment after PCR amplification, using the oligonucleotides of SEQ ID NO's 39 and 40, from the plasmid pHELY::A1 (WO04106347) and cloned into the *Eco*RI*-Kpn*I sites of pDESTR4R3NphleoN, yielding plasmid pDESTR4R3NphleoNamdS. This plasmid was linearized with *Not*l and transfected to *Penicillium chrysogenum* protoplasts (produced according to standard protocols, *i.e*. Cantoral et al., 1987, Biotechnology 5: 494-497; Swinkels et al., 1997, WO97/06261) however Glucanex^{™} (Sigma) was applied as lysing enzyme. After transfection the protoplasts were plated out on selective regeneration agar plates with phleomycin (on mineral medium agar as in US 2002/0039758, without phenyl acetic acid but supplemented with 1M Saccharose, 15 g/L agar to solidify and 50 mg/L phleomycin). After sporulation the absence of the *amdS* gene was checked on acetamide and fluoroacetamide agar plates (WO9706261). A final check was done with PCR, using two specific primer sets which should give only fragments in mutants with a targeted deletion (Fig. 6). The integration at the left flank was checked by using oligonucleotides SEQ ID NO's 41 and 42, while the integration at the right flank was checked by using oligonucleotides SEQ ID NO's 43 and 44. All phleomycin resistant and *amdS* non-utilizing colonies were shown to have gene targeting towards the niaD locus. For the WT only one in 95 phleomycin resistant colonies was also *amdS* non-utilizing. So by using a combination of strains with an improved HR/NHR ratio and a second DNA fragment which can be used as a selectable marker the gene targeting efficiency is increased from 1.1% (WT) to 100% (for both *hdfA* and *hdfB* mutants). From both experiments an isolate with the correct *niaD* deletion was purified, called HTAN and HTBN, respectively. These results show the beneficial effect of combining strains with improved HR/NHR ratios and the use of a second selectable marker. The gene targeting frequency in WT strains is around 1% (example 3). Strains with improved HR/NHR ratios, but transformed without a second selectable marker show 47-56% correct gene targeting (example 3). Strains with normal HR/NHR ratios (*i.e*. WT strains), but transformed with a second selectable marker show 16-63% correct gene targeting (examples 1 and 2). However, when using strains with improved HR/NHR ratios and a second selectable marker surprisingly a 100% correct gene targeting is obtained (this example).

### Example 5

### Improved homologous recombination frequencies in hdfB mutant strains of Penicillium chrysogenum using a second selectable marker

A second example of very efficient gene targeting by combining the effect strains with an optimized frequency of homologous recombination and the use of a second selectable marker was obtained by using two constructs named pDONR201 *amdS*ni800 and pDONR201*amdS*ni1200 (Fig. 7). As a recipient strain the HTBN strain of example 4 was used. The 441 bp central part of the *niaD* gene lacking in strain HTBN was restored via, and only via, exact gene targeting. Hereto, the restoring *niaD* fragment was flanked by either 800 (as in pDONR201amdSni800) or 1200 bp (as in pDONR201amdSni1200) homologous targeting sequences. Also, here the *amdS* expression cassette was used as second selectable marker outside the homologous flanking regions. First, the *amdS* expression cassette was PCR amplified from pHELY-A1 and cloned via Gateway technology into the pDONR201 vector, yielding plasmid pDONR201-amdS (Table 5). Second, the restoring *niaD* fragments of 2041 (=800+441+800) and 2841 (=1200+441+1200) were obtained after PCR amplification from genomic DNA (Table 5), digested with *Mlu*I and *Not*l*,* and cloned into pDONR201amdS, yielding plasmids pDONR201amdSni800 and pDONR201amdSni1200, respectively.

**Table 5. Oligonucleotides used for PCR amplification.**

| Fragment | Template | Forward oligonucleotid e | Reverse oligonucleotid e | Cloned in vector |
|---|---|---|---|---|
| PgpdA*-amdS* | pHELY-A1 | SEQ ID NO 45 | SEQ ID NO 46 | pDONR™201 |
| 2041 bp *niaD* | Genomic DNA | SEQ ID NO 47 | SEQ ID NO 48 | pDONR201*amdS* |
| 2841 bp *niaD* | Genomic DNA | SEQ ID NO 49 | SEQ ID NO 50 | pDONR201*amdS* |

The vectors pDONR201*amdS*ni800 and pDONR201*amdS*ni1200 were linearized with either Mlul or Nrul, and transfected to *Penicillium chrysogenum* protoplasts (produced according to standard protocols, *i.e*. Cantoral et al., 1987, Biotechnology 5: 494-497; Swinkels et al., 1997, WO97/06261) however Glucanex^{™} (Sigma) was applied as lysing enzyme. After transfection the protoplasts were plated out on selective regeneration agar plates with nitrate as the sole nitrogen source (per liter: NaCl, 3.0 g; KH₂PO₄, 1.0 g; FeSO₄.7H20, 10.0 mg; MgSO₄.7H₂O, 0.5 mg; Glucose, 10.0 g; Saccharose, 342.0 g; Oxoid agar, 15.0 g; NaNO₃, 1 g; 1 M phosphate buffer pH6,8, 10 ml). Eleven transformants were obtained (see Table 6) and these were transferred to fresh plates, without saccharose to induce sporulation. After sporulation the absence of the *amdS* gene was checked on acetamide agar plates (see WO9706261) and none of the transformants was able to grow, showing that no non-homologous recombination occurred and only 100% correct gene targeting was obtained. The results clearly show the beneficial effect of combining strains with improved HR/NHR ratios and the use of a second selectable marker. The gene targeting frequency in WT strains is around 1% (see example 3). Strains with improved HR/NHR ratios, but transformed without a second selectable marker show 47-56% correct gene targeting (see example 3). Strains with normal HR/NHR ratios (*i.e*. WT strains), but transformed with a second selectable marker show 16-63% correct gene targeting (examples 1 and 2). However, when using strains with improved HR/NHR ratios and a second selectable marker surprisingly a 100% correct gene targeting is obtained (this example).

**Table 6. Transformants obtained after restoration of the 441 bp central niaD fragment in strain HTBN.**

| Plasmid | Transformants from Mlul digest | Transformants from Nrul digest |
|---|---|---|
| Water | 0 | 0 |
| pDONR201 *amdS*ni800 | 2 | 1 |
| pDONR201*amdS*ni1200 | 4 | 4 |

## Claims

1. An in-vitro method to construct eukaryotic cells having a target sequence in a chromosomal DNA sequence replaced by a desired replacement sequence, comprising:
modifying a parent eukaryotic cell with a preference for NHR to provide a eukaryotic cell having an increased HR/NHR ratio as compared to the parent cell,
providing a DNA molecule comprising a first DNA fragment comprising a desired replacement sequence flanked at its 5' and 3' sides by DNA sequences substantially homologous to sequences of the chromosomal DNA flanking the target sequence and a second DNA fragment comprising an expression cassette comprising a gene encoding a selection marker and regulatory sequences functional in the eukaryotic cell operably linked thereto;
transforming the modified eukaryotic cells with the DNA molecule;
growing the cells to obtain transformed progeny cells having the DNA molecule inserted into the chromosome,
deselecting the progeny cells in which the DNA molecule is inserted in the chromosome via a non-homologous recombination event by expression of the second marker; and
obtaining cells wherein the target sequence in the chromosomal DNA sequence is replaced by the desired replacement sequence,
wherein the substantially homologous DNA sequences flanking the replacement sequence have a degree of identity to a chromosomal DNA sequence flanking the target sequence of at least 80% over a region of not more than 3 kb.

2. The method according to claim 1, wherein the parent eukaryotic cell with a preference for NHR is a eukaryotic cell having a HR/NHR ratio ≤ 0.5, preferably ≤ 0.2, more preferably ≤ 0.1, most preferably ≤ 0.05.

3. The method according to claim 1or 2, wherein the HR/NHR ratio is increased at least 2 times, preferably at least 4 times.

4. The method according to any one of the claim 1-3, wherein modifying the parent eukaryotic cell is achieved by increasing the efficiency of the HR pathway and/or decreasing the efficiency of the NHR pathway.

5. The method according to any one of the claims 1-4, wherein modifying the parent eukaryotic cell is achieved by down regulating, preferably inactivating, equivalents of the yeast genes *KU70, KU80, RAD50, MRE11, XRS2, LIG4, LIF1, NEJ1* and/or *SIR4,* involved in the NHR pathway

6. The method according to any one of the claims 1-5 wherein the replacement sequence comprises a selection marker, a modified version of the target sequence and/or additional copies of a sequence of interest being present in the genome of the eukaryotic cell.

7. The method according to any one of the claims 1-6, wherein the regulatory sequences of the selection marker expression cassette are heterologous to the fungal cell.

8. The method according to any one of the claims 1-7, wherein the regulatory sequences of the selection marker expression cassette comprise a constitutive or an inducible promoter.

9. The method according to any one of the claims 1-8, wherein the selection marker is a toxin.

10. The method according to any one of the claims 1-8, wherein the selection marker is a conditional selection marker.

11. The method according to any one of the claims 1-10, wherein the selection marker is diphtheria toxin A encoded by the *dtA* gene, nitrate reductase encoded by the *niaD* gene, orotidine-5'-monophosphate decarboxylase encoded by the *pyrG* gene, acetamidase encoded by the *amdS* gene, or thymidine kinase encoded by the *tk* gene.

## Patentansprüche

1. In-vitro-Verfahren für die Konstruktion von eukaryontischen Zellen, bei denen eine Zielsequenz in einer chromosomalen DNA-Sequenz durch eine gewünschte Ersatzsequenz ersetzt ist, das die folgenden Schritte umfasst:
Modifizieren einer eukaryontischen Elternzelle mit Bevorzugung für NHR, wodurch man zu einer eukaryontischen Zelle mit einem erhöhten HR/NHR-Verhältnis verglichen mit der Elternzelle gelangt,
Bereitstellen eines DNA-Moleküls, das Folgendes umfasst: ein erstes DNA-Fragment, umfassend eine gewünschte Ersatzsequenz, die 5'-seitig und 3'-seitig von DNA-Sequenzen, die zu Sequenzen der chromosomalen DNA, die die Zielsequenz flankiert, im Wesentlichen homolog sind, flankiert ist, und ein zweites DNA-Fragment, umfassend eine Expressionskassette, die ein Gen, das für einen Selektionsmarker und Regulationssequenzen, die in der eukaryontischen Zelle in operativer Verknüpfung funktionell sind, umfasst;
Transformieren der modifizierten eukaryontischen Zellen mit dem DNA-Molekül;
Heranziehen der Zellen, um zu transformierten Nachkommenschaftszellen, die das DNA-Molekül in das Chromosom insertiert enthalten, zu gelangen,
Deselektieren der Nachkommenschaftszellen, bei denen das DNA-Molekül in das Chromosom über ein nichthomologes Rekombinations-Event insertiert ist, durch Expression des zweiten Markers; und
Gewinnen von Zellen, bei denen die Zielsequenz in der chromosomalen DNA-Sequenz durch die gewünschte Ersatzsequenz ersetzt ist,
wobei die die Ersatzsequenz flankierenden, im Wesentlichen homologen DNA-Sequenzen eine Identität zu einer chromosomalen DNA-Sequenz, die die Zielsequenz flankiert, in einem Ausmaß von mindestens 80% über eine Region von nicht mehr als 3 kB aufweisen.

2. Verfahren nach Anspruch 1, wobei es sich bei der eukaryontischen Elternzelle mit Bevorzugung für NHR um eine eukaryontische Zelle mit einem HR/NHR-Verhältnis von ≤0,5, vorzugsweise ≤ 0,2, stärker bevorzugt ≤0,1, am stärksten bevorzugt ≤ 0,05 handelt.

3. Verfahren nach Anspruch 1 oder 2, wobei das HR/NHR-Verhältnis mindestens um das 2fache, vorzugsweise um das 4fache, erhöht ist.

4. Verfahren nach einem der Ansprüche 1-3, wobei das Modifizieren der eukaryontischen Elternzelle **dadurch** erfolgt, dass man die Effizienz des HR-Wegs erhöht und/oder die Effizienz des NHR-Wegs erniedrigt.

5. Verfahren nach einem der Ansprüche 1-4, wobei das Modifizieren der eukaryontischen Elternzelle durch Herunterregulieren, vorzugsweise Inaktivieren, von Äquivalenten der Hefegene *KU70, KU80, RAD50, MRE11, XRS2, LIG4, LIF1, NEJ1* und/oder *SIR4,* die am NHR-Weg beteiligt sind, erfolgt.

6. Verfahren nach einem der Ansprüche 1-5, wobei die Ersatzsequenz einen Selektionsmarker, eine modifizierte Version der Zielsequenz und/oder zusätzliche Kopien von einer interessierenden Sequenz, die im Genom der eukaryontischen Zelle vorliegt, umfasst.

7. Verfahren nach einem der Ansprüche 1-6, wobei die Regulationssequenzen der Selektionsmarkerexpressionskassette heterolog im Bezug auf die pilzliche Zelle sind.

8. Verfahren nach einem der Ansprüche 1-7, wobei die Regulationssequenzen der Selektionsmarkerexpressionskassette einen konstitutiven oder einen induzierbaren Promoter umfassen.

9. Verfahren nach einem der Ansprüche 1-8, wobei es sich bei dem Selektionsmarker um ein Toxin handelt.

10. Verfahren nach einem der Ansprüche 1-8, wobei es sich bei dem Selektionsmarker um einen bedingten Selektionsmarker handelt.

11. Verfahren nach einem der Ansprüche 1-10, wobei es sich bei dem Selektionsmarker um das von dem dtA-Gen codierte Diphtherie-Toxin A, um die von dem *niaD*-Gen codierte Nitratreduktase, um die von dem *pyrG*-Gen codierte Orotidin-5'-monophosphatde-carboxylase, um die von dem amdS-Gen codierte Acetamidase oder um die von dem *tk*-Gen codierte Thymidinkinase handelt.

## Revendications

1. Procédé in vitro de construction de cellules eucaryotes dans une séquence d'ADN chromosomique de laquelle une séquence cible est remplacée par une séquence de remplacement souhaitée, comprenant :
la modification d'une cellule eucaryote parente ayant une préférence pour la NHR, de façon à fournir une cellule eucaryote présentant un rapport HR/NHR augmenté par comparaison avec celui de la cellule parente ;
la fourniture d'une molécule d'ADN comprenant un premier fragment d'ADN comprenant une séquence de remplacement souhaitée, flanquée sur ses côtés 5' et 3' par des séquences d'ADN sensiblement homologues de séquences de l'ADN chromosomique flanquant la séquence cible, et un deuxième fragment d'ADN comprenant une cassette d'expression qui comprend un gène codant pour un marqueur de sélection, et des séquences régulatrices fonctionnelles dans la cellule eucaryote et qui y sont liées d'une manière opérationnelle ;
la transformation des cellules eucaryotes modifiées avec la molécule d'ADN ;
la croissance des cellules, pour obtenir des cellules descendantes transformées, dans lesquelles la molécule d'ADN est insérée dans le chromosome ;
la désélection des cellules descendantes dans lesquelles la molécule d'ADN est insérée dans le chromosome par un événement de recombinaison non homologue par expression du deuxième marqueur ; et
l'obtention de cellules dans lesquelles la séquence cible se trouvant dans la séquence d'ADN chromosomique est remplacée par la séquence de remplacement souhaitée, les séquences d'ADN sensiblement homologues qui flanquent la séquence de remplacement ayant avec la séquence d'ADN chromosomique qui flanque la séquence cible un degré d'identité d'au moins 80 %, sur une région non supérieure à 3 kb.

2. Procédé selon la revendication 1, dans lequel la cellule eucaryote parente ayant une préférence pour la NHR est une cellule eucaryote ayant un rapport HR/NHR ≤ 0,5, de préférence ≤ 0,2, plus particulièrement ≤ 0,1, tout spécialement ≤ 0,05.

3. Procédé selon la revendication 1 ou 2, dans lequel le rapport HR/NHR est multiplié par au moins 2, de préférence par au moins 4.

4. Procédé selon l'une quelconque des revendications 1 à 3, dans lequel la modification de la cellule eucaryote parente est réalisée par augmentation de l'efficacité de la voie de la HR et/ou par une diminution de l'efficacité de la voie de la NHR.

5. Procédé selon l'une quelconque des revendications 1 à 4, dans lequel la modification de la cellule eucaryote parente est réalisée par une régulation à la baisse, de préférence par une inactivation d'équivalents des gènes de levure *KU70, KU80, RAD50, MRE11, XRS2, LIG4, LIF1, NEJ1* et/ou *SIR4* impliqués dans la voie de la NHR.

6. Procédé selon l'une quelconque des revendications 1 à 5, dans lequel la séquence de remplacement comprend un marqueur de sélection, une version modifiée de la séquence cible et/ou des copies additionnelles d'une séquence d'intérêt, présente dans le génome de la cellule eucaryote.

7. Procédé selon l'une quelconque des revendications 1 à 6, dans lequel les séquences régulatrices de la cassette d'expression du marqueur de sélection sont hétérologues vis-à-vis de la cellule fongique.

8. Procédé selon l'une quelconque des revendications 1 à 7, dans lequel les séquences régulatrices de la cassette d'expression du marqueur de sélection comprennent un promoteur constitutif ou un promoteur inductible.

9. Procédé selon l'une quelconque des revendications 1 à 8, dans lequel le marqueur de sélection est une toxine.

10. Procédé selon l'une quelconque des revendications 1 à 8, dans lequel le marqueur de sélection est un marqueur de sélection conditionnel.

11. Procédé selon l'une quelconque des revendications 1 à 10, dans lequel le marqueur de sélection est la toxine diphtérique A codée par le gène *dtA*, la nitrate réductase codée par le gène *niaD,* l'orotidine-5'-monophosphate décarboxylase codée par le gène *pyrG,* l'acétamidase codée par le gène *amdS,* ou la thymidine kinase codée par le gène *tk*.
